# EUROPÄISCHE PATENTSCHRIFT

(11) **EP 1 466 167 B1**
(45) Veröffentlichungstag und Bekanntmachung des Hinweises auf die Patenterteilung: **19.07.2006**
(21) Anmeldenummer: 02747168.9
(22) Anmeldetag: 24.05.2002
(51) Int. Cl.: G01N 27/12, G01N 30/00, G01N 33/00

(54) **VERFAHREN UND DETEKTOR ZUR ERFASSUNG VON GASEN**
METHOD AND DETECTOR FOR DETECTING GASES
PROCEDE ET DETECTEUR POUR LA DETECTION DE GAZ

(30) Priorität: 25.05.2001 DE 10125837
(43) Veröffentlichungstag der Anmeldung: 13.10.2004
(73) Patentinhaber: AIRSENSE Analytics GmbH, 19061 Schwerin (DE)
(72) Erfinder: WALTE, Andreas, 19055 Schwerin (DE); MÜNCHMEYER, Wolf, 19055 Schwerin (DE)
(74) Vertreter: Jaap, Reinhard
(86) Internationale Anmeldenummer: PCT/DE2002/001889
(87) Internationale Veröffentlichungsnummer: WO 2002/095389

(56) Entgegenhaltungen:
- EP-A- 0 829 718
- WO-A-00/62046
- DE-C- 19 807 658
- PATENT ABSTRACTS OF JAPAN vol. 007, no. 238 (P-231), 22. Oktober 1983 (1983-10-22) & JP 58 124939 A (HITACHI SEISAKUSHO KK), 25. Juli 1983 (1983-07-25)

## Beschreibung

Die Erfindung betrifft ein Verfahren nach dem Oberbegriff des Anspruches 1 und einen entsprechenden Detektor nach dem Oberbegriff des Anspruches 4.

Solche Verfahren und Detektoren werden zur Identifizierung von Einzelverbindungen in Gemischen als auch zur Identifizierung der Gemische eingesetzt. Anwendungen finden sich in der Umwelttechnik, Sicherheitstechnik, wie z.B. zur Erfassung von Leckagen in der Industrie oder z.B. zur Schwelbranderkennung, in der Lebensmittelindustrie, in der medizinischen Diagnostik, als auch in der chemischen Industrie zur Qualitätskontrolle.
Es ist inzwischen von großer Bedeutung, dass beispielsweise Einzelstoffe oder die Summe einzelner Verbindungen mit bestimmten Eigenschaften, wie z.B. Qualität des Lebensmittels, korrelieren.
Diese Zustände können teilweise mit Detektoren, welche in Form von einzelnen Gassensoren oder in einer Kombination aus verschiedenen Gassensoren auftreten, erfaßt werden. Die Meßsignale der einzelnen Gassensoren können dann mit vorher gemessenen, bzw. auch gespeicherten Signalen verglichen werden und der gemessene Zustand beschrieben werden.
Solche Verfahren sind seit längerem bekannt. Einige dieser Systeme, bei denen mehrere Gassensoren mit Querempfindlichkeit in Form von Gassensorenarrays eingesetzt werden, sind seit einigen Jahren unter dem Namen "elektronische Nasen" bekannt. Diese Geräte bestehen aus einer Anordnung aus mehren Gassensoren, beispielsweise die "kalten" Gassensoren, wie Schwingquarze, bzw. leitfähige Polymere oder die "heißen" Gassensoren, wie Halbleitergassensoren und einer Ansteuer- und eine Auswerteelektronik, bzw. Auswerterechner. Die "kalten" Gassensoren werden bei Umgebungstemperaturen eingesetzt, während die "heißen" Gassensoren typischerweise bei Arbeitstemperaturen des Gassensors zwischen T=200°C und T=600°C eingesetzt werden.

Aus DE-2313413A1 ist weiterhin bekannt, dass das Wechseln der Arbeitstemperatur der heißen Gassensoren den zusätzlichen Vorteil hat, dass dabei auch die Selektivität der Gassensoren geändert wird.

Nachteilig ist, dass in vielen Anwendungen die Selektivität der Gassensoren nicht ausreicht, so dass sie z.B. auf Verbindungen, bzw. Gase welche in hohen Konzentrationen auftreten, aber für die Problemstellung nicht relevant sind, reagieren. Dies kann z.B. Feuchte oder Ozon in der Umgebungsluft, oder Methan bei der Erfassung von Gerüchen in der Umwelttechnik, bis hin zu Ethanol in alkoholischen Getränken sein. Eine Erhöhung der Anzahl der Gassensoren führt dabei nicht immer zu einer Verbesserung der Auftrennung.
Nachteilig ist, dass sehr häufig, wie z.B. bei Gerüchen, die Nachweisgrenze der Gassensoren zu gering ist. Ein weiterer Nachteil ist, dass bei den einfachen Gassensoren auch Sensordrift auftritt, welche sich negativ bei der Reproduzierbarkeit und Vergleichbarkeit zwischen Gassensoren gleichen Typs auswirkt.
Bei den heißen Gassensoren gibt es zusätzlich Schwierigkeiten bei Einsätzen in der chemischen Industrie, da dort häufig auch der Explosionsschutz zu berücksichtigen ist.

Aus W. Muenchmeyer et al. (2000), Sensors and Actuators B69, 379-383 und DE 198 07 658 C1 ist bekannt, dass durch Verwendung von Adsorbentien, welche als Granulat in Röhrchen gefüllt werden, eine selektive Anreicherung möglich ist. Das Gasgemisch wird hierzu über ein Adsorbens-Granulat, wie z.B. spezielle Polymere (Tenax®) oder Kohlenstoff basierte Adsorbentien, geleitet. Durch Wechselwirkungen mit dem Adsorbens werden bei den erwähnten Adsorbentien die mittel- und schwerflüchtigen Verbindungen gesammelt, während die leichtflüchtigen durch das Adsorbens geleitet werden. Üblicherweise wird das Meßgas mit einer Gaspumpe über das Adsorbens gefördert. Mittels einer Erwärmung können nach einer definierten Sammelzeit die Substanzen wieder freigesetzt und mit einem Detektor, bzw. Sensoranordnung nachgewiesen werden. Über andere Adsorbentien, wie z.B. Zeolithe, Aktivkohle oder Silikate lassen sich auch kleinere und leichtflüchtigere Verbindungen anreichern.
Nachteilig ist, dass bei letzteren schwerflüchtige Substanzen auch angereichert werden, aber mittels thermischer Desorption nicht freigesetzt werden können und die
Eigenschaften des Adsorbens verändern.
Aus EP 0 055 624 A1 ist bekannt, dass die Adsorbentien auch in Form einer Membran hergestellt werden können. Nach der Anreicherungsphase wird die Membran beheizt und mit einem nachfolgendem Detektor, in diesem Fall ein Massenspektrometer, werden die freigesetzten Verbindungen nachgewiesen.
Nachteilig bei den Verfahren ist, dass Anreicherung und Gassensoren physikalisch getrennt sind und normalerweise zwei Geräte aufgebaut werden, so dass z.B. komplexe Gaswege mit z.T. mehreren Pumpen und umfangreiche Elektronik, welche mit entsprechend hoher elektrischer Leistung versorgt werden muß, notwendig sind. Nachteilig ist außerdem, dass bei hoher Staubbelastung das Adsorbens verschmutzt und die Eigenschaften des Adsorbens sich somit verändern.
Aus der US PAT. No. 5,783,154 ist bekannt, dass heiße Metalloxidsensoren auch mit dünnen Siliziumschichten beschichtet werden, um somit über Diffussionseffekte die
Selektivität des Sensors zu beeinflussen.
Nachteilig ist, dass durch den direkten Kontakt mit dem Sensor keine Anreicherung möglich ist, und die Selektivität in erster Linie über die Geometrie der Moleküle gesteuert wird. Polymere können wegen der hohen Temperatur der Sensoren hier nicht eingesetzt werden.
Weiterhin ist aus JP 58124939 A und Motorola, "MGS1100 Carbon Monoxide Gas Sensor", Motorola Semiconductor Technical Data, MGS1100/D (1997) bekannt, dass durch Kopplung eines heißen Sensors mit porösen Schichten aus Silicagel, Zeolithen und Kalzium Chlorid, bzw. bei Motorola einem Aktivkohlefilter, die Selektivität beeinffusst werden kann. So ist z.B. bei Motorola die Querempfindlichkeit eines CO Sensors gegenüber flüchtigen organischen Verbindungen verringert worden. Nachteilig ist, dass der Sensor nicht gereinigt wird und die porösen Partikel, bzw. der Aktivkohlefilter nach einer gewissen Zeit nicht mehr filtern und die nicht interessierenden Substanzen zum Sensor gelingen, welche Fehlmessungen auslösen. Hohe

### Ersatzblatt

Konzentrationen von Lösungsmitteln, Zigarettenrauch oder Ammoniak sind deshalb laut Datenblatt von Motorola zu vermeiden.

Bei den Schwingquarzsensoren (kalter Gassensor) ist bekannt, dass es verschiedene Wechselwirkungen der zu messenden Substanzen mit dem Polymer gibt, welches als Adsorbens auf dem Schwingquarz aufgebracht wird.
Nachteilig sind die langen Zeiten, welche zur Desorption, bzw. Reinigung der sensitiven Schichten (Polymere) benötigt werden, falls schwerflüchtige
Verbindungen auf den Schichten auskondensieren.
Aus P. Boecker et al. (2000), Sensors and Actuators B70, 37-42 ist bekannt, dass durch einen Temperaturwechselbetrieb der kalten Sensoren die Desorption der Substanzen beschleunigt wird und somit auch die Meßzyklen verkürzt werden. Nachteilig ist, dass durch den direkten Kontakt mit dem Sensor dann auch der Sensor erwärmt wird. Da bei diesem Typ von Sensor das Meßsignal sehr stark von der Temperatur abhängig ist, wird ein zweiter Sensor benötigt, um die Änderung der Temperatur zu berücksichtigen.
Aus der WO 00/62046 ist bekannt, dass die Empfindlichkeit von Metalloxidsensoren verändert werden kann, wenn eine Membran aus porösem Silizium vor Dünnfilm-Metalloxidsensoren positioniert wird. Durch Beschichtung der Membran mit katalytisch wirkenden Substanzen kann neben einer Anregung der chemischen Verbindungen auch eine chemische Umsetzung erfolgen, was den Vorteil hat, dass insbesondere bei Dünnfilmsensoren die Nachweisgrenzen verbessert werden können. Nachteilig ist, dass bei porösen Dickfilmsensoren keine Verbesserung der Nachweisgrenze beobachtet wird und dass mit Silizium-Membranen keine Anreicherung von organischen Verbindungen möglich ist.

Es besteht also die Aufgabe ein Verfahren und Einrichtung zu entwickeln, welche die Nachweisgrenze der Detektoren verbessert und gleichzeitig die Selektivität, d.h. den Nachweis einzelner Verbindungen bei gleichzeitiger Anwesenheit anderer Verbindungen in wesentlich höheren Konzentrationen, steigert. Zusätzlich besteht Ersatzblatt die Notwendigkeit die Sensordrift und dabei auch die Lebensdauer der Gassensoren durch einen Schutz vor Stäuben und Aerosolen zu verbessern.

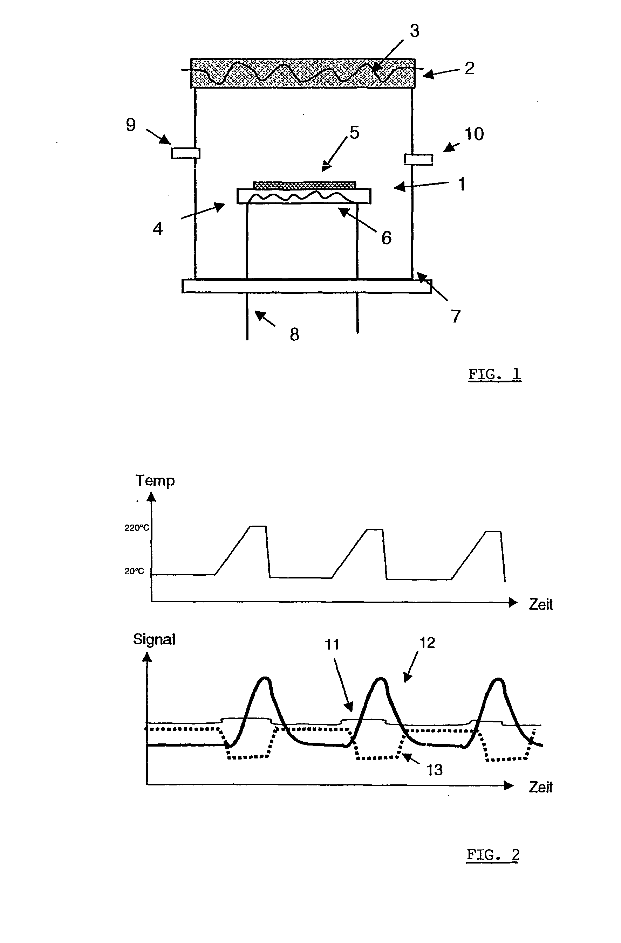

Die Erfindung wird in den unabhängigen Ansprüchen 1 und 4 definiert und soll beispielhaft anhand einer schematischen Darstellung mit einem Metalloxid-Sensor in Fig. 1 näher erläutert werden. Dazu zeigen
Fig. 1: Detektor mit Flachmembran
Fig. 2: Von Temperatureffekten korrigierte Messsignale des Detektors.

Die Einrichtung zur Durchführung des Verfahrens zur Bestimmung gasförmiger Verbindungen besteht in der Hauptsache aus einem Detektor 1, welcher aus einer Kombination von einem Adsorbens 2, welches optional mit einer Möglichkeit zur thermischen Erwärmung, d.h. einem Heizelement 3 vorgesehen ist, und einem Gassensor 4 besteht. Der Gassensor 4 besitzt elektrische Zuleitungen zum Auslesen des Meßsignals von der gassensitiven Schicht 5 und auch zur Energieversorgung des Gassensors. Weiterhin ist das Trägersubstrat mit einem Heizer 6 für den Gassensor und das Gehäuse des Detektors 7 dargestellt. Die notwendigen elektrischen Anschlüsse 8 und ein optionaler Einlaß für Spülgas 9 bzw. Auslaß 10 sind zusätzlich dargestellt.
Das Adsorbens ist zweckmäßigerweise in Form einer Membran gestaltet, welche den Gassensor 4 umhüllt ohne diesen zu berühren. Als Membranmaterial werden Polymere, wie z.B. Silikone, Fluorelastomere, oder Tenaxmembranen, welche im kalten Zustand mittel- und schwerflüchtige Verbindungen anreichern können, eingesetzt. Über eine thermische Desorption, d.h. eine Erwärmung der Membran, lassen sich die angereicherten Verbindungen wieder freisetzen. Durch einen Temperaturwechselbetrieb, d.h. die kalte Membran zum Anreichern und anschließend die warme Membran zum Freisetzen der Verbindungen, wird die Selektivität des Gassensors gesteigert. Die desorbierten Verbindungen müssen durch einen Lösungs- Diffusionsprozess, von der Messgasseite durch das Adsorbens in Form einer Membran zum Detektor gelangen.
Mit der Membran wird die Nachweisgrenze verbessert, gleichzeitig dient sie aber auch zum Schutz des Detektors, da störende Partikel oder Partikel bildende Substanzen nicht auf den Detektor gelangen können. Zusätzlich lassen sich die Explosionsschutzeigenschaften einiger Detektoren, insbesondere der heißen Sensoren verbessern.
Um die Selektivität der Adsorbentien in Form von Membranen zu ändern, ist es zweckmäßig auch Membranen, welche mit anderen Adsorbentien gefüllt werden, wie z.B. Tenax®, Kohlenstoff basierte Adsorbentien, Zeolithen, Calixarene, usw. einsetzen. Bei Verwendung von Elastomeren wie Silikon oder Viton als Membranmaterial wird auch die Lebensdauer des Adsorbens verbessert, da durch die glatte Membran keine Partikel in die Poren der Granulate des Adsorbens gelangen und diese auch nicht verstopfen können. Zusätzlich wird verhindert, dass schwerflüchtige Verbindungen mit geringer Diffusionsrate durch die Membran, welche mit den eingesetzten Füllstoffen (Adsorbentien) auch nicht thermisch zu desorbieren sind, auf das Adsorbens gelingen.
Bei der Verwendung von heißen Gassensoren, wie z.B. MOS, Mosfet oder Peltistoren ist eine direkte Beheizung der Adsorbentien nicht immer notwendig, da durch einen Temperaturwechselbetrieb des Gassensors auch gleichzeitig diese durch Wärmetransport, wie Konvektion oder Diffusion erwärmt wird.

Diese hat insbesondere bei den oben erwähnten Gassensoren den Vorteil, dass bei kalter Membran, wie z.B. einer Silikonmembran mit Tenax Füllung, nur kleine Moleküle, wie z.B. H2, CO, CH4 gemessen werden, während bei größeren Temperaturen auch mittel- und schwerflüchtige Verbindungen erfaßt werden.
In Fig.2 sind die Meßsignale eines Detektors bei Temperaturwechselbetrieb beispielsweise dargestellt. Das von reinen Temperatureffekten korrigierte Messsignal eines MOS Gassensors ist bei einer zyklisch beheizten Membran als Funktion der Zeit dargestellt. Es wird das Messignal eines Gemisches aus leicht- und schwerflüchtigen Verbindungen, mit und ohne Dosierung von Referenzluft während der thermischen Desorption, dargestellt. Während die Membran kalt ist, werden nur leichtflüchtige Verbindungen gemessen. Falls nur leichtflüchtige Verbindungen vorhanden
sind, hat die Erhöhung der Temperatur der Membran nur einen geringen Einfluß auf das Meßsignal. Das Detektorsignal bei leichtflüchtigen Verbindungen 11 zeichnet sich durch eine nicht sehr ausgeprägte Temperaturabhängigkeit des Messsignals des Detektors aus.
Das Detektorsignal bei Anwesenheit von mittel- bis schwerflüchtigen Verbindungen 12 zeigt deutliche Signalanstiege während der Erwärmung, da diese Verbindungen jetzt freigesetzt und besser durch die Membran gelangen. Falls mittel- und schwerflüchtige Verbindungen nicht erfaßt werden sollen, kann während der Erwärmung der Membran auch der Raum zwischen Membran und Sensor mit sauberer Luft gespült werden. Die Kurve des Detektorsignals für leichtflüchtige Verbindungen bei Spülung mit Nullluft während der Desorption 13 unterscheidet sich deutlich von den Ergebnissen ohne Spülung, da die angereicherten Verbindungen wenn überhaupt nur in verdünnter Form zum Gassensor gelangen.

Über die Wahl des Füllmaterials der Membran lassen sich die Nachweisgrenzen für einige Verbindungen erheblich verbessern. So können z.B. Zeolithe oder "Nanoröhrchen" aus Kohlenstoff eingesetzt werden um selektiv auch kleinere Moleküle, bzw. Permanentgase bis hin zu Wasserstoff anzureichern. Neben der Steigerung der Nachweisgrenze, der Verbesserung der Selektivität, lassen sich durch den beschriebenen Aufbau auch die Produktionskosten erheblich verringern.
Insbesondere die Schutzwirkung der Membran, bezüglich der Kontamination mit Partikeln, Flüssigkeiten oder auch der Beeinflussung durch Luftströmung, kommen hier zum tragen. Durch die Schutzwirkung kann der Detektor auch in sehr staubiger Umgebung eingesetzt werden, wie z.B. zur Gasmessung in Abgasen, oder als Brandmelder. Analysen von Flüssigkeiten, z.B. Lösemittel in Wasser, lassen sich auch mit dem Detektor durchführen, insbesondere wenn während der Ausheizphase der Membran die Flüssigkeit entfernt wird.
Weiterhin ist es zweckmäßig über Kombination dieser Gassensoren und der Membranen, z.B. unterschiedliche Membranen bei einem Gassensor oder bei einer Membran mit einer Anordnung von Gassensoren, Meßsysteme für verschiedene Anwendungen zu realisieren. Die Detektoren lassen sich in einer Sensorkammer mit Probenahmesystem integrieren, oder auch direkt in den Prozeß einsetzen.

## Patentansprüche

1. Verfahren zur Bestimmung von gasförmigen Verbindungen in Staub belasteter Luft mit einem Detektor (1), bei dem eine gasförmige Verbindung einem kalten oder beheizbaren Gassensor (4), durch Überbrückung eines Abstandes zwischen einem Adsorbens (2) und dem Gassensor (4) zugeleitet, und gemessen wird,
**dadurch gekennzeichnet, dass** vor der Messung des Gassensors (4) die gasförmige Verbindung durch einen membranförmigen Adsorbens (2) physikalisch vom Gassensor (4) getrennt wird, im kalten Zustand auf dem Adsorbens (2) angereichert wird und durch die Erwärmung des Gassensors (4) im heißen Zustand von der Messgasseite durch den Adsorbens (2) in Richtung des Gassensors (4) wieder freigesetzt wird, wobei das Adsorbens (2) durch einen Temperaturwechselbetrieb des Gassensors (4) zyklisch erwärmt wird.

2. Verfahren nach Anspruch 1,
**dadurch gekennzeichnet, dass** der Temperaturwechselbetrieb des Gassensors (4) zur zyklischen Erwärmung des Adsorbens (2) durch ein direkt auf dem Adsorbens (2) wirkendes Heizelement (3) erfolgt.

3. Verfahren nach Anspruch 1,
**dadurch gekennzeichnet, dass** das Adsorbens (2) von der Messgasseite oder auch von der Seite des Gassensors (4) durch eine Desorption mit sauberer Luft bzw. einer definierten Referenzluft gasförmigen Verbindungen aus der Absorbens (2) entfernt werden, um somit nur die vorher adsorbierten chemischen Verbindungen mittels der Diffusion durch das Adsorbens (2) zum Gassensor (4) hin zu erfassen.

4. Detektor zur Bestimmung von gasförmigen Verbindungen, bestehend aus einem beheizbaren Gassensor (4) mit einer gassensitiven Schicht (5) zur Aufnahme der gasförmigen Verbindung, mit elektrischen Anschlüssen (8) zur Verarbeitung des Messsignals und mit einem Heizer (6) zur Erwärmung des Gassensors (4), wobei der Gassensor (4) von einer Membran umschlossen ist, ohne ihn zu berühren,
**dadurch gekennzeichnet, dass** die Membran als ein Adsorbens (2) ausgebildet ist, welches das Messgas vom Gassensor (4) trennt, im kalten Zustand anreichert und im erwärmten Zustand in Richtung des Gassensors (4) freisetzt, wobei zwischen dem Gassensor (4) und dem Adsorbens (2) ein Abstand ist und dieser Abstand so groß ist, dass einerseits die freigesetzte gasförmige Verbindung den Gassensor (4) erreicht und anderseits die Wärme des Gassensors (4) auf den Adsorbens (2) abstrahlt, und der Heizer (6) mit Einrichtungen zur zyklischen Erwärmung des Gassensors (4) ausgestattet ist.

5. Detektor nach Anspruch 4,
**dadurch gekennzeichnet, dass** ein den Gassensor (4) umgebendes und den Absorbens (2) einfassendes Gehäuse (7) Ein- und Auslässe (9, 10) für Spülgas zum Spülen des Zwischenraumes zwischen dem Adsorbens (2) und dem Gassensor (4) vorgesehen sind.

6. Detektor nach Anspruch 4,
**dadurch gekennzeichnet, dass** das Adsorbens (2) zusätzlich mit einem Heizelement (3) zur zyklischen Erwärmung des Adsorbens (2) ausgestattet ist.

7. Detektor nach Anspruch 4,
**dadurch gekennzeichnet, dass** eine Anordnung mehrerer Gassensoren (4) hinter einem Adsorbens (2) positioniert ist.

8. Detektor nach Anspruch 4,
**dadurch gekennzeichnet, dass** eine Anordnung mehrerer Adsorbentien (2) vor einem Gassensor (4) positioniert ist.

9. Verwendung eines Detektors (1) nach Anspruch 8,
**dadurch gekennzeichnet, dass** der Detektor (1) die Adsorbentien (2) zu unterschiedüchen Zeiten ausheizt, um somit eine lückenlose Überwachung zu ermöglichen.

## Claims

1. Method of detecting gaseous compounds in air entraining dust by means of a detector (1), whereby a gaseous compound is fed to and measured at a gas sensor (4), which is cold or can be heated, by bridging a distance between an adsorbent (2) and the gas sensor (4), **characterised in that** prior to the gas sensor (4) taking the measurement, the gaseous compound is physically separated from the gas sensor (4) by a membrane-shaped adsorbent (2), concentrated on the adsorbent (2) in the cold state and, by heating the gas sensor (4), released from the measurement gas side through the adsorbent (2) in the direction of the gas sensor (4) again in the hot state, and the adsorbent (2) is heated in cycles by operating the gas sensor (4) at alternating temperatures.

2. Method as claimed in claim 1,
**characterised in that** the gas sensor (4) is operated at alternating temperatures to heat the adsorbent (2) in cycles by means of a heating element (3) acting directly on the adsorbent (2) .

3. Method as claimed in claim 1,
**characterised in that** adsorbent (2) from the measurement gas side or also from the side of the gas sensor (4) is removed from the adsorbent (2) by means of desorption with clean air or a defined reference air of gaseous compounds so that only the previously adsorbed chemical compounds are detected by means of diffusion through the adsorbent (2) towards the gas sensor (4).

4. Detector for detecting gaseous compounds comprising a heatable gas sensor (4) with a gas-sensitive layer (5) for absorbing the gaseous compound, with electric terminals (8) for processing the measurement signal and a heater (6) for heating the gas sensor (4), and the gas sensor (4) is surrounded by a membrane but is not in contact with it, **characterised in that** the membrane is provided in the form of an adsorbent (2) which separates the measurement gas from the gas sensor (4) , concentrating it in the cold state and releasing it in the direction of the gas sensor (4) in the heated state, and a distance exists between the gas sensor (4) and the adsorbent (2) and the size of this distance is such that the released gaseous compound reaches the gas sensor (4) on the one hand and the heat of the gas sensor (4) irradiates the adsorbent (2) on the other hand, and the heater (6) is equipped with devices for heating the gas sensor (4) in cycles.

5. Detector as claimed in claim 4,
**characterised in that** a housing (7) surrounding the gas sensor (4) and incorporating the adsorbent (2) is provided with inlets and outlets (9, 10) for flushing gas for flushing the intermediate space between the adsorbent (2) and the gas sensor (4) .

6. Detector as claimed in claim 4,
**characterised in that** the adsorbent (2) is additionally equipped with a heating element (3) for heating the adsorbent (2) in cycles.

7. Detector as claimed in claim 4,
**characterised in that** an array of several gas sensors (4) is positioned behind an adsorbent (2).

8. Detector as claimed in claim 4,
**characterised in that** an array of several adsorbents (2) is positioned in front of a gas sensor (4).

9. Use of a detector (1) as claimed in claim 8,
**characterised in that** the detector (1) heats the adsorbents (2) at different times, thereby enabling gap-free monitoring.

## Revendications

1. Procédé pour déterminer des composés gazeux dans des poussières d'air pollué comprenant un détecteur (1), en amenant un composé gazeux vers un capteur de gaz (4) froid ou chauffé avec pontage d'une distance entre un adsorbant (2) et le capteur de gaz (4), pour le mesurer,
**caractérisé en ce qu'**
avant la mesure du capteur de gaz (4) le composé gazeux est séparé physiquement du capteur de gaz (4) par un adsorbant (2) en forme de membrane, enrichi à l'état froid sur l'adsorbant (2) puis dégagé à l'état chaud à travers l'adsorbant (2), suite au chauffage du capteur de gaz (4), depuis le côté gaz de mesure en direction du capteur de gaz (2), l'adsorbant (2) étant chauffé cycliquement par un fonctionnement de changement de température du capteur de gaz (4).

2. Procédé selon la revendication 1,
**caractérisé en ce que**
le fonctionnement de changement de température du capteur de gaz (4) pour le chauffage cyclique de l'adsorbant (2) est assuré par un élément chauffant (3) agissant directement sur l'adsorbant (2).

3. Procédé selon la revendication 1,
**caractérisé en ce que**
on sépare l'adsorbant (2) du côté gaz de mesure ou encore du côté du capteur de gaz (4) on supprime des composés gazeux de l'adsorbant (2) par une désorption avec de l'air propre ou un air de référence défini, pour ainsi ne détecter que les composés chimiques adsorbés préalablement au moyen de la diffusion à travers l'adsorbant (2) vers le capteur de gaz (4).

4. Détecteur pour déterminer des composés gazeux, comprenant un capteur de gaz (4) pouvant être chauffé muni d'une couche sensible aux gaz (5) pour recevoir le composé gazeux, des raccords électriques (8) pour le traitement du signal de mesure, et un système de chauffage (6) pour chauffer le capteur de gaz (4), le capteur de gaz (4) étant entouré par une membrane sans contact avec celui-ci,
**caractérisé en ce que**
la membrane est un adsorbant (2) qui sépare le gaz de mesure du capteur de gaz (4), l'enrichit à l'état froid et le dégage à l'état chauffé en direction du capteur de gaz (4), avec entre le capteur de gaz (4) et l'adsorbant (2) une distance suffisamment grande pour que d'une part le composé gazeux dégagé arrive au capteur de gaz (4) et d'autre part la chaleur du capteur de gaz (4) rayonne sur l'adsorbant (2), et le système de chauffage (6) est muni de dispositifs pour le chauffage cyclique du capteur de gaz (4).

5. Détecteur selon la revendication 4,
**caractérisé en ce qu'**
un boîtier (7) entourant le capteur de gaz (4) et l'adsorbant (2) comporte des entrées et des sorties (9, 10) d'un gaz de rinçage pour rincer l'espace intermédiaire entre l'adsorbant (2) et le capteur de gaz (4).

6. Détecteur selon la revendication 4,
**caractérisé en ce que**
l'adsorbant (2) est en plus muni d'un élément chauffant (3) pour le chauffage cyclique de adsorbant (2).

7. Détecteur selon la revendication 4,
**caractérisé en ce qu'**
un ensemble de plusieurs capteurs de gaz (4) est positionné derrière un adsorbant (2).

8. Détecteur selon la revendication 4,
**caractérisé en ce qu'**
un ensemble de plusieurs adsorbants (2) est positionné devant un capteur de gaz (4).

9. Utilisation d'un détecteur (1) selon la revendication 8,
**caractérisé en ce que**
le détecteur (1) chauffe les adsorbants (2) à différents moments pour ainsi permettre une surveillance sans faille.
